# FASCICULE DE BREVET EUROPEEN

(11) **EP 2 271 623 B1**
(45) Date de publication et mention de la délivrance du brevet: **24.07.2013**
(21) Numéro de dépôt: 09750028.4
(22) Date de dépôt: 28.04.2009
(51) Int. Cl.: C07D 213/79, C07F 9/38, C07C 409/24, A62D 3/36

(54) **COMPOSITION D'ACIDE PERPROPIONIQUE STABLE EN MILIEU BASIQUE**
IN EINEM BASISCHEN MEDIUM STABILE PERPROPIONSÄUREZUSAMMENSETZUNG
PERPROPIONIC ACID COMPOSITION THAT IS STABLE IN A BASIC MEDIUM

(30) Priorité: 29.04.2008 FR 0852895
(43) Date de publication de la demande: 12.01.2011
(73) Titulaire: Arkema France, 92700 Colombes (FR)
(72) Inventeur: COUTURIER, Jean-Luc, F-69006 Lyon (FR); KERVENNAL, Jacques, F-69005 Lyon (FR); GOFFINET, Pierre Charles, F-91190 Gif-sur-Yvette (FR); LION, Claude, F-75116 Paris (FR)
(74) Mandataire: Killis, Andréas
(86) Numéro de dépôt international: PCT/FR2009/050778
(87) Numéro de publication internationale: WO 2009/141547

(56) Documents cités:
- EP-A- 0 864 562
- WO-A-91/13058

## Description

La présente invention concerne des compositions d'acide perpropionique stables en milieu basique, un procédé de stabilisation de l'acide perpropionique, ainsi que l'utilisation d'une combinaison spécifique de composés pour stabiliser l'acide perpropionique en milieu basique.

On sait que les solutions d'acide perpropionique, préparées à partir de peroxyde d'hydrogène et d'acide propionique, obéissent à une loi d'équilibre dont l'équation peut être schématisée de la manière suivante

CH₃ -CH₂-CO₂H + H₂O₂ < -> CH₃-CH₂-CO₃H + H₂O

La cinétique d'évolution de ce système est relativement lente ; elle est accélérée par des acides forts tels que l'acide sulfurique ou l'acide phosphorique qui jouent, en l'occurrence, le rôle de catalyseur. La présence de ces acides forts permet au système d'évoluer plus rapidement jusqu'à son état d'équilibre. Toutefois, il est connu qu'une fois parvenu à son état d'équilibre, le système perd lentement l'oxygène actif qu'il contient, cette perte se traduisant par une lente décroissance de la teneur en peracide et en peroxyde d'hydrogène. Pour pallier ce phénomène, des stabilisants comme l'acide dipicolinique sont utilisés.

Certaines utilisations de l'acide perpropionique nécessitent la mise en oeuvre de solutions d'acide perpropionique en milieu basique. Il s'agit par exemple de l'utilisation de compositions à base d'acide perpropionique pour la décontamination de produits toxiques organophosphorés ou organosoufrés, par exemple les agents toxiques utilisés dans le domaine militaire ou les produits phytosanitaires utilisés dans le domaine agricole.

La demande internationale WO01/30452, décrit une composition basique comprenant de l'acide perpropionique, et de l'acide dipicolinique utilisé comme stabilisant. L'utilisation d'une telle composition pour décontaminer des produits toxiques est décrite dans ce document. Néanmoins, une telle composition ne présente pas une stabilité satisfaisante. Il existe donc un besoin pour de nouvelles compositions d'acide perpropionique, plus stables en milieu basique.

Les inventeurs ont donc mis au point une nouvelle composition à base d'acide perpropionique. Selon l'invention, cette composition comprend de l'acide perpropionique, de l'acide dipicolinique et de l'acide hydroxyéthylidène diphosphonique (HEDP).

De manière surprenante on a constaté que la composition telle que définie ci-dessus est très stable, et notamment beaucoup plus stable que des compositions comprenant de l'acide perpropionique, des séquestrants organiques différents de l'HEDP, tels que le DETMP de sodium (sel de l'acide diéthylènetriamine pentaméthylènephosphonique) ou l'EDTMP de sodium (sel de l'acide éthylènediamine tétraméthylènephosphonique) traditionnellement utilisés comme stabilisants de H₂O₂. En effet, ces séquestrants organiques ne confèrent aucun avantage en terme de stabilisation de la composition décontaminante au contraire de l'HEDP (exemple 2)

Avantageusement, l'acide perpropionique utilisé se présente sous la forme d'une solution aqueuse, en particulier telle qu'elle a été obtenue par réaction d'une solution aqueuse de peroxyde d'hydrogène avec l'acide propionique, en présence d'un catalyseur, tel que l'acide sulfurique ou l'acide borique. Un tel procédé de préparation est par exemple décrit dans la demande de brevet français FR2462425. Contrairement aux solutions anhydres d'acide perpropionique décrites dans les demandes de brevets français n° FR2464947 et FR2519634, les solutions aqueuses sont obtenues avec un procédé simple, ne mettant pas en oeuvre une étape de distillation azéotropique avec des solvants organiques toxiques ou inflammables tels que le 1,2-dichloroéthane ou le cyclohexane. EP 0864 562 décrit la stabilisation des solutions aqueuses des peracides, en présence d'un stabilisant à base d'étain, obtenu à partir d'un sel d'étain et d'un sel phosphoré. WO 91/13058 décrit la préparation de solutions diluées stables de peracides aliphatiques inférieurs et plus particulièrement d'acide peracétique, pour utilisation de désinfection industrielle ou d'hygiène personnelle ou domestique.

Ainsi, l'acide perpropionique utilisé se présente sous la forme d'une solution aqueuse comprenant : de l'acide perpropionique comme produit, de l'acide propionique comme réactif n'ayant pas réagi, de l'eau oxygénée comme réactif n'ayant pas réagi, de l'acide sulfurique ou de l'acide borique comme catalyseur.

La composition objet de l'invention peut donc comprendre :
- de l'acide perpropionique,
- de l'acide dipicolinique,
- de l'acide hydroxyéthylidène diphosphonique
- de l'acide propionique,
- de l'eau oxygénée, et
- de l'acide sulfurique, et/ou de l'acide borique.

Les compositions définies ci-dessus sont des solutions qui ne forment qu'une seule phase dans l'eau, ce qui permet une mise en oeuvre aisée de la composition objet de l'invention dans une solution aqueuse.

A titre d'exemple, l'acide hydroxyéthylidène diphosphonique de la composition objet de l'invention peut être constitué du produit commercialisé par la société SOLUTIA sous la dénomination commerciale DEQUEST 2010®.

Dans un mode de réalisation préféré, la composition objet de l'invention comprend, en poids par rapport au poids total de la composition :
- 30 à 40 % d'acide perpropionique,
- 0,05 à 2 % d'acide dipicolinique, et
- 0,5 à 5 % d'acide hydroxyéthylidène diphosphonique

Lorsque la composition objet de l'invention est diluée dans une solution aqueuse basique, la solution obtenue est stable, comme cela est illustré dans l'exemple 3.

Dans un mode de réalisation particulier de l'invention, la composition définie ci-dessus se présente sous la forme d'une solution aqueuse basique. Ainsi, l'invention a également pour objet une solution aqueuse basique comprenant une composition telle que définie ci-dessus.

Par « solution aqueuse basique », on entend au sens de la présente invention toute solution comprenant de l'eau et dont le pH est supérieur à 7.

Avantageusement, les solutions aqueuses basiques selon l'invention ont un pH compris entre 8 et 12, et de manière encore plus préférée entre 8,5 et 9,5. Si nécessaire, le pH peut être ajusté à l'aide d'un agent alcalinisant. Ainsi, dans un mode de réalisation de l'invention, la solution aqueuse basique comprend un agent alcalinisant. Cet agent alcalinisant peut être choisi parmi l'hydroxyde de sodium, l'hydroxyde de potassium, l'hydroxyde d'ammonium, le carbonate ou l'hydrogénocarbonate de sodium, le carbonate ou l'hydrogénocarbonate de potassium, le phosphate de sodium, le phosphate de potassium, le phosphate d'ammonium, les silicates de sodium, les silicates de potassium, les silicates d'ammonium, les borates de sodium, les borates de potassium, les borates d'ammonium, et leurs mélanges.

Lorsque la composition objet de l'invention est diluée dans une solution aqueuse basique, l'acide dipicolinique, l'acide perpropionique et l'HEDP peuvent se présenter sous la forme de leurs sels correspondants.

La stabilité de la composition est définie par une perte non significative de concentration en acide perpropionique après 12 mois de stockage à température en milieu acide, c'est-à-dire que la concentration en acide perpropionique n'est pas inférieure à 90% de la concentration initiale. La composition selon l'invention présente également une perte de la concentration en acide perpropionique après 1 heure en milieu basique, moins importante que la perte de la concentration en acide perpropionique mesurée pour une composition non conforme à l'invention (Exemple 3). Typiquement l'efficacité de la formulation en décontamination n'est pas inférieure à 80% de l'activité initiale.

Les compositions selon l'invention sont de préférence mises en oeuvre en solution aqueuse à une concentration de 0,5 à 40 %, et de préférence de 3 à 10% en poids d'acide perpropionique par rapport au poids total de la solution. Elles peuvent également être mises en oeuvre sous forme d'émulsions ou de microémulsions après dispersion dans un solvant organique non miscible à l'eau tel que par exemple les hydrocarbures aliphatiques ou aromatiques, éventuellement chlorés, par exemple le toluène, le xylène, le chlorure de méthylène et le tétrachloroéthylène.

Une solution aqueuse selon l'invention peut comprendre, outre la composition objet de l'invention, un composé choisi parmi un tampon alcalin, un agent tensioactif, et leurs mélanges.

De telles solutions aqueuses sont particulièrement bien adaptées à une utilisation en tant qu'agent décontaminant destiné à détruire des composés organophosphorés ou organosoufrés.

Par "tampon alcalin ", on entend au sens de la présente invention aussi bien un seul type de tampon alcalin qu'un mélange de tampons alcalins. Les compositions selon l'invention comprennent un tampon alcalin tel que un carbonate alcalin ou un silicate alcalin, notamment le carbonate de potassium ou de sodium.

Par "agent tensioactif', on entend au sens de la présente invention un agent tensioactif anionique, cationique, non ionique ou amphotère, utilisé à raison de 0 à 25%, de préférence 5 à 20% en poids par rapport au poids total de la composition.

En tant qu'agent tensioactif cationique on peut citer ceux du type ammonium quaternaire, et par exemple
- le bromure de cétyltriméthyl ammonium
- le chlorure de cétyltriméthyl ammonium
- le bromure de cétyldiméthyl hydroxy-2 éthyl ammonium
- le bromure de cétylméthyl bis (hydroxy-2 éthyl) ammonium
- le bromure de benzyltriméthyl ammonium, et
- le bromure de cétyl diaza-1,4-bicyclo [2.2.2] octylammonium.

Ces agents tensio-actifs sont connus et, pour la plupart, disponibles dans le commerce. Ils peuvent être préparés par les méthodes décrites par C.A. Bunton et al., J. Am. Chem. Soc., 95,2912 (1973) et par L. Horner et al., Phosphorus and Sulfur, 11,339 (1981).

En tant qu'agent tensioactif non ionique utilisable de manière optionnelle on peut citer par exemple :
- les alcools gras alcoxylés de formule : R-[(CH₂)₂O]ₘ-[ CH₂CH(CH₃)O]ₙ-H dans laquelle R est un alkyle ou alcényle comprenant 8 à 22 atomes de carbone, de préférence 12 à 14 atomes de carbone ; n et m, indépendamment, représentent un nombre entier de 0 à 50, dont la somme n+m > 1;
- les alkylpolyglucosides de formule R"'-[glucose]ₙ- dans laquelle R"' est un alkyle en C₈-C₁₆ et n est un nombre entier de 1 à 3;
- les esters de sucres réduits, les esters de polyols et leurs dérivés éthoxylés, tels que par exemple le monostéarate de sorbitan, de glycérol ou d'éthylèneglycol, ou le monostéarate de sorbitan éthoxylé à 20 oxydes d'éthylène;
- les alkylamides éthoxylées de formule: 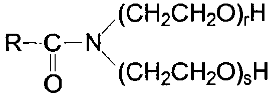 dans laquelle R est tel que défini ci-dessus et r et s sont des nombres entiers de 0 à 15, dont la somme r+ s> 1;
- les alkylpyrrolidones, dont le groupement alkyle est en C₆-C₂₀; et leurs mélanges.

De préférence, l'acide perpropionique est présent dans ladite solution aqueuse à une concentration par exemple de 0,05 à 4,5 mole/L.

Les solutions objet de l'invention peuvent également comprendre au moins un constituant choisi parmi un agent hydrotrope, tel que l'urée, le cumène sulfonate de sodium, un agent viscosant tel que la gomme xanthane, l'amidon de maïs modifié, l'hydroxyéthylcellulose, ou encore un agent antimousse.

La présente invention a également pour objet un procédé de stabilisation d'une solution aqueuse basique comprenant de l'acide perpropionique caractérisé en ce qu'il comprend une étape de dissolution d'acide hydroxyéthylidène diphosphonique ou de l'un de ses sels dans ladite solution d'acide perpropionique.

Dans le procédé défini ci-dessus, la solution aqueuse basique peut comprendre en outre de l'acide dipicolinique.

Dans le procédé décrit ci-dessus, l'acide perpropionique mis en oeuvre peut avoir été obtenu par réaction d'une solution aqueuse de peroxyde d'hydrogène avec de l'acide propionique, en présence d'un catalyseur, tel que l'acide sulfurique ou l'acide borique.

La présente invention concerne également l'utilisation d'acide hydroxyéthylidène diphosphonique et/ou l'un de ses sels pour stabiliser une solution aqueuse basique comprenant de l'acide perpropionique et éventuellement de l'acide dipicolinique.

A titre d'exemple, le sel de l'acide hydroxyéthylidène diphosphonique peut être un sel de sodium ou de potassium. Lorsque de l'hydroxyéthylidène diphosphonate de sodium est utilisé, celui-ci peut être constitué du produit commercialisé par la société SOLUTIA sous la dénomination commerciale DEQUEST 2016®.

Les exemples suivants illustrent la présente invention sans toutefois en limiter la portée. Dans ces exemples, les pourcentages sont en poids sauf indication contraire.

### EXEMPLE 1

Dans un réacteur de 3 litres, on charge 1000g d'acide propionique, 15g d'acide sulfurique, 7,5g d'acide dipicolinique et 25g d'acide hydroxyéthylidène diphosphonique à 60% (Dequest 2010, SOLUTIA). A température ambiante et sous agitation, on ajoute 500g d'eau oxygénée 70% sur une période de 10 minutes. On laisse 12h sous agitation.

La quantité d'oxygène actif est dosée par iodométrie (réaction avec KI et dosage au thiosulfate de sodium). L'eau oxygénée est dosée avec du sulfate de cérium(IV). La concentration en acide perpropionique est déduite par différence entre l'oxygène actif total et l'eau oxygénée. La méthode de dosage de l'oxygène actif au thiosulfate est citée dans l'ouvrage : Organic Peroxides de D. Swern Volume 1 (Wiley SBN 471839604), Chapitre 1.

La composition obtenue est :

| C1 | % poids |
|---|---|
| Acide perpropionique | 38,2 |
| Eau oxygénée | 8,3 |
| Acide propionique | 33,8 |
| Eau | 17,2 |
| Acide sulfurique | 1,0 |
| Acide dipicolinique | 0,5 |
| Acide hydroxyéthylidène diphosphonique | 1,0 |

### EXEMPLE 2 (non conforme à l'invention)

3 compositions non conformes à l'invention ont été préparées selon l'exemple 1 : en
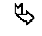 en supprimant l'acide hydroxyéthylidène diphosphonique (composition C2)
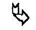 en remplaçant l'acide hydroxyéthylidène diphosphonique par une quantité égale de diéthylènetriamine pentaméthylphosphonate de sodium (Dequest 2066) (composition C3)
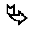 en remplaçant l'acide hydroxyéthylidène diphosphonique par une quantité égale d'éthylènediamine tétraméthylphosphonate de sodium (Dequest 2046) (composition C4)

### EXEMPLE 3

Les compositions préparées dans les exemples 1 et 2 sont amenées à pH basique par ajout d'une solution aqueuse de potasse à 20% et de carbonate de potassium (ratio massique K₂CO₃/KOH=2/1)

La concentration en acide perpropionique est mesurée par la méthode décrite dans l'exemple 1.

| Composition | pH | Concentration en acide perpropionique en % poids après 5 minutes |
|---|---|---|
| C1 | 8,6 | 2,5 |
| C2 | 9,1 | 2,1 |
| C3 | 8,6 | 1,1 |
| C4 | 8,0 | 0,7 |

Ces exemples illustrent l'avantage de l'utilisation d'une combinaison d'acide dipicolinique et d'acide hydroxyéthylidène phosphonique par rapport à l'acide dipicolinique seul ou associé à d'autres acides phosphoniques.

## Revendications

1. Solution aqueuse comprenant une composition comprenant :
(i) de l'acide perpropionique,
(ii) de l'acide dipicolinique, et
(iii) de l'acide hydroxyéthylidène diphosphonique
avec ladite solution aqueuse comprenant un agent alcalinisant et présentant un pH supérieur à 7, et de préférence compris entre 8 et 12, et de manière encore plus préférée entre 8,5 et 9,5.

2. Solution aqueuse selon la revendication 1, **caractérisée en ce que** ladite composition comprend, en poids par rapport au poids total de la composition :
- 30 à 40 % d'acide perpropionique,
- 0,05 à 2 % d'acide dipicolinique,
- 0,5 à 5 % d'acide hydroxyéthylidène diphosphonique.

3. Solution selon l'une des revendications 1 ou 2, **caractérisée en ce que** l'acide perpropionique est présent à une concentration de 0,05 à 4,5 mole/L.

4. Procédé de stabilisation d'une solution aqueuse basique comprenant de l'acide perpropionique et de l'acide dipicolinique, **caractérisé en ce qu'**il comprend une étape de dissolution d'acide hydroxyéthylidène diphosphonique ou de l'un de ses sels dans ladite solution d'acide perpropionique.

5. Utilisation d'acide hydroxyéthylidène diphosphonique ou de l'un de ses sels pour stabiliser une solution aqueuse basique comprenant de l'acide perpropionique et de l'acide dipicolinique.

## Patentansprüche

1. Wässrige Lösung, umfassend eine Zusammensetzung, umfassend:
(i) Perpropionsäure,
(ii) Dipicolinsäure und
(iii) Hydroxyethylidendiphosphonsäure,
wobei die wässrige Lösung ein Alkalinisierungsmittel umfasst und einen pH-Wert von mehr als 7 und vorzugsweise zwischen 8 und 12 und noch weiter bevorzugt zwischen 8,5 und 9,5 aufweist.

2. Wässrige Lösung nach Anspruch 1, **dadurch gekennzeichnet, dass** die Zusammensetzung, bezogen auf das Gesamtgewicht der Zusammensetzung:
- 30 bis 40 Gew.-% Perpropionsäure,
- 0,05 bis 2 Gew.-% Dipicolinsäure,
- 0,05 bis 5 Gew.-% Hydroxyethylidendiphosphonsäure
umfasst.

3. Lösung nach einem der Ansprüche 1 oder 2, **dadurch gekennzeichnet, dass** die Perpropionsäure in einer Konzentration von 0,05 bis 4,5 mol/L vorliegt.

4. Verfahren zur Stabilisierung einer basischen wässrigen Lösung, die Perpropionsäure und Dipicolinsäure umfasst, **dadurch gekennzeichnet, dass** man in der Perpropionsäurelösung Hydroxyethylidendiphosphonsäure oder ein Salz davon löst.

5. Verwendung von Hydroxyethylidendiphosphonsäure oder eines Salzes davon zur Stabilisierung einer basischen wässrigen Lösung, die Perpropionsäure und Dipicolinsäure umfasst.

## Claims

1. Aqueous solution comprising a composition comprising:
(i) perpropionic acid,
(ii) dipicolinic acid, and
(iii) hydroxyethylidene diphosphonic acid with the said aqueous solution comprising a basifying agent and having a pH above 7, and preferably between 8 and 12, and even more preferably between 8.5 and 9.5.

2. The aqueous solution as claimed in claim 1, **characterized in that** the said composition comprises, by weight relative to the total weight of the composition:
- 30% to 40% of perpropionic acid,
- 0.05% to 2% of dipicolinic acid,
- 0.5% to 5% of hydroxyethylidene diphosphonic acid.

3. The solution as claimed in any one of claims 1 or 2, **characterized in that** the perpropionic acid is present at a concentration of from 0.05 to 4.5 mol/1.

4. A method for stabilizing a basic aqueous solution comprising perpropionic acid and dipicolinic acid, **characterized in that** it comprises a step of dissolving hydroxyethylidene diphosphonic acid, or any of its salts, in said perpropionic acid solution.

5. The use of hydroxyethylethylidene diphosphonic acid, or any of its salts, for stabilizing a basic aqueous solution comprising perpropionic acid and dipicolinic acid.
